# EUROPEAN PATENT APPLICATION

(11) **EP 3 441 012 A2**
(43) Date of publication of application: **13.02.2019**
(21) Application number: 18188435.4
(22) Date of filing: 10.08.2018
(51) Int. Cl.: A61B 17/072, A61N 5/10

(54) **BRACHYTHERAPY STAPLE CAPTURE BUTTRESS**

(30) Priority: 11.08.2017 US 201762544037 P; 17.07.2018 US 201816037229
(71) Applicant: Covidien LP, Mansfield, MA 02048 (US)
(72) Inventor: TAYLOR, Joseph J., Newtown, CT 06470 (US)
(74) Representative: Maschio, Antonio

(57) **Abstract**

A method of stapling tissue includes clamping tissue between a cartridge assembly and an anvil assembly of a loading unit, firing the loading unit such that the fasteners secured in the cartridge assembly pass through a body portion of a first buttress that is positioned between the clamped tissue and one of the cartridge assembly or the anvil assembly, trimming a first portion of the first buttress that is adjacent a first edge of the tissue, and removing the first portion of the first buttress from a surgical site. The first buttress capturing fasteners that are fired from a portion of the cartridge assembly which extends beyond the tissue. The first portion of the first buttress including at least one remnant fastener.

## Description

### CROSS-REFERENCE TO RELATED APPLICATIONS

This application claims the benefit of and priority to U.S. Provisional Patent Application Serial No. 62/544,037 filed August 11, 2017, the entire disclosure of which is incorporated by reference herein.

### BACKGROUND

### 1. Technical Field

The present disclosure relates to surgical instruments and, more specifically, staple retention systems for surgical stapling instruments.

### 2. Discussion of Related Art

Surgical staplers are used during surgical procedures to join segments of transected tissue and/or organs. During some surgical procedures, a surgical stapler is used to join tissue and/or organs that do not extend the full length of a stapler cartridge of the surgical stapler. In these situations, "remnant" staples, which are staples not deployed into tissue and/or organs, may fall into a body cavity or surrounding tissue. When traditional staples are used, these remnant staples are not a concern.

During some surgical procedures, e.g., brachytherapy, radioactive staples can be deployed to treat tissue and/or organs in which the radioactive staples are deployed. In these procedures, it may be preferable to avoid leaving "remnant" staples in a body cavity adjacent tissue and/or organs unintended for radiotherapy. It will be appreciated that manually retrieving individual "remnant" staples would be burdensome, time consuming, and/or impossible.

Accordingly, there is a need for a surgical stapler that does not leave "remnant" staples within a body cavity after a surgical procedure.

### SUMMARY

In an aspect of the present disclosure, a method of stapling tissue includes clamping tissue between a cartridge assembly and an anvil assembly of a loading unit, firing the loading unit such that the fasteners secured in the cartridge assembly pass through a body portion of a first buttress that is positioned between the clamped tissue and one of the cartridge assembly or the anvil assembly, trimming a first portion of the first buttress that is adjacent a first edge of the tissue, and removing the first portion of the first buttress from a surgical site. The first buttress captures fasteners that are fired from a portion of the cartridge assembly which extends beyond the tissue. The first portion of the first buttress includes at least one remnant fastener.

In aspects, trimming the first portion of the first buttress adjacent the first edge of the tissue includes trimming the first portion of the first buttress adjacent a distal end of the first buttress or a proximal end of the first buttress.

In some aspects, the method includes trimming a second portion of the first buttress adjacent a second edge of the tissue which is adjacent a proximal end of the first buttress and removing the second portion of the first buttress from the surgical site which includes at least one remnant fastener.

In certain aspects, firing the loading unit includes the fasteners piercing a body portion of a second buttress which is positioned between the clamped tissue and the anvil assembly while the first buttress is positioned between the clamped tissue and the cartridge assembly. The method may include trimming a first portion of the second buttress that is adjacent the first edge of the tissue.

In another aspect of the present disclosure, a loading unit includes a cartridge assembly, an anvil assembly, a first buttress, and a sled. The cartridge assembly has a first tissue contacting surface and fasteners which are disposed therein. The anvil assembly has a second tissue contacting surface that opposes the first tissue contacting surface. The cartridge and anvil assemblies have a first position in which the first and second tissue contacting surfaces are spaced apart from one another and a second position in which at least one of the first and second tissue contacting surfaces are approximated relative to the other to clamp tissue therebetween. The first buttress is operatively secured to one of the first or second tissue contacting surfaces. The sled is translatable through the cartridge assembly to fire the fasteners from the cartridge assembly towards the anvil assembly. The fasteners are configured to pass through the first buttress and form upon contact with the anvil. The first buttress is configured to capture at least one of the formed fasteners.

In aspects, the first buttress is configured to capture each formed fastener. The sled may be configured to sequentially fire the fasteners. The fasteners may be radioactive fasteners such that the fasteners are configured to emit energy to tissue.

In some aspects, the first buttress is operatively secured to the first tissue contacting surface. The loading unit may include a second buttress that is operatively secured to the second tissue contacting surface. The fasteners may be configured to pass through the second buttress and form upon contact with the anvil. The second buttress may be configured to capture each formed fastener. The first buttress may be secured to the first tissue contacting surface.

In another aspect of the present disclosure, a surgical instrument includes a handle assembly, an elongate body, and a loading unit. The elongate body extends from the handle assembly. The loading unit is supported by a distal portion of the elongate body. The loading unit includes a cartridge assembly, an anvil assembly, a first buttress, and a sled. The cartridge assembly has a first tissue contacting surface and fasteners disposed therein. The anvil assembly has a second tissue contacting surface. The cartridge and anvil assemblies have a first position in which the first and second contacting surfaces are spaced apart from one another and a second position in which at least one of the first and second tissue contacting surfaces are approximated relative to the other to clamp tissue therebetween. The first buttress is operatively secured to one of the first or second tissue contacting surfaces. The sled is translatable through the cartridge assembly to fire the fasteners from the cartridge assembly towards the anvil assembly. The fasteners are configured to pass through the first buttress and form upon contact with the anvil. The first buttress is configured to capture at least one of the formed fasteners. The handle assembly may be manually actuated.

Further, to the extent consistent, any of the aspects described herein may be used in conjunction with any or all of the other aspects described herein.

### BRIEF DESCRIPTION OF THE DRAWINGS

Various aspects of the present disclosure are described hereinbelow with reference to the drawings, which are incorporated in and constitute a part of this specification, wherein:
FIG. 1 is a perspective view of a surgical instrument including a loading unit provided in accordance with the present disclosure;
FIG. 2 is an exploded view, with parts separated, of the loading unit of FIG. 1;
FIG. 3 is a top view of a buttress of the loading unit of FIG. 2;
FIG. 4 is a perspective view of tissue disposed between jaws of the loading unit of the surgical instrument of FIG. 1 with the jaws in a spaced-apart position, the loading unit including a buttress;
FIG. 5 is a perspective view of the tissue of FIG. 4 with a buttress secured to the tissue with fasteners deployed in the tissue and the buttress;
FIG. 6 is a perspective view of the buttress of FIG. 5 with portions of the buttress severed adjacent the tissue to remove captured "remnant" staples; and
FIG. 7 is a schematic view of a "telesurgery" system provided in accordance with the present disclosure.

### DETAILED DESCRIPTION

Embodiments of the present disclosure are now described in detail with reference to the drawings in which like reference numerals designate identical or corresponding elements in each of the several views. As used herein, the term "clinician" refers to a doctor, a nurse, or any other care provider and may include support personnel. Throughout this description, the term "proximal" refers to the portion of the device or component thereof that is closest to the clinician and the term "distal" refers to the portion of the device or component thereof that is farthest from the clinician.

This disclosure relates generally to a buttress for loading units of surgical instruments that are configured to capture "remnant" staples during a surgical procedure to prevent the "remnant" staples from remaining within a body cavity of a patient.

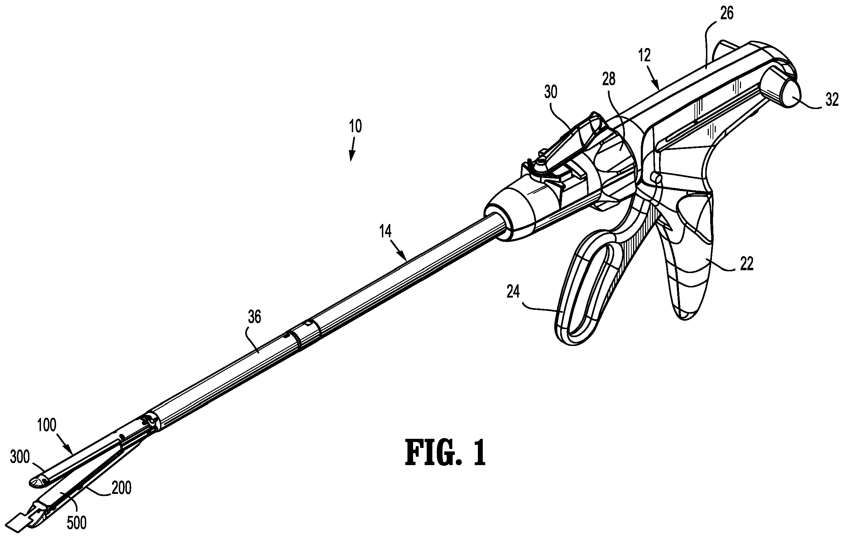

Referring now to FIG. 1 there is disclosed a surgical stapling instrument, generally referred to as 10. In the interest of brevity, this disclosure will focus primarily on a buttress utilized in a loading unit 100, e.g., a single use loading unit ("SULU") or a disposable loading unit ("DLU"). For simplicity, hereinafter, SULU or DLU will be referred to as "DLU," but it should be understood to include either or both a DLU or SULU.

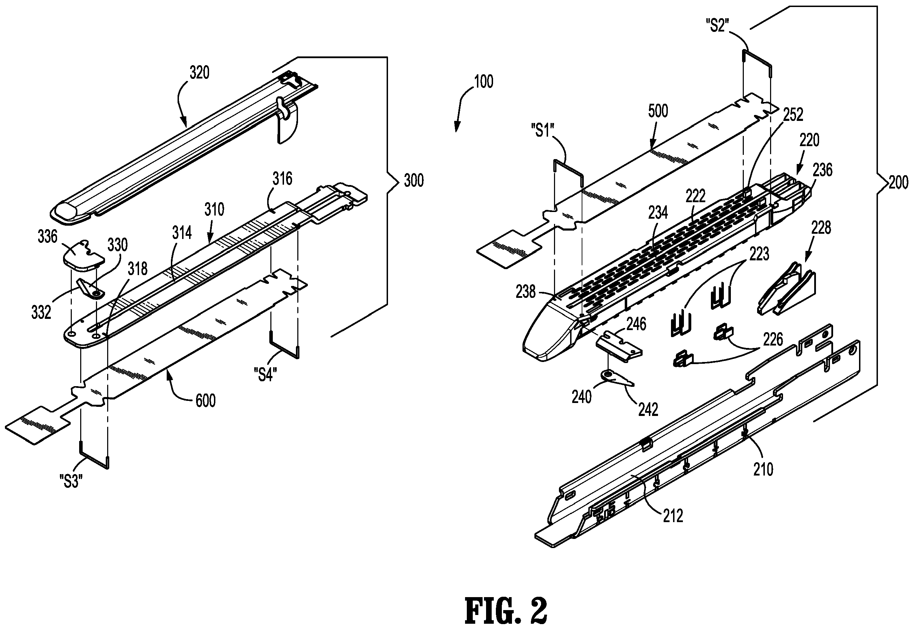

The surgical stapling instrument 10 generally includes a handle assembly 12 and an elongate body 14 extending distally from the handle assembly 12. A DLU 100 is releasably secured to a distal portion of the elongate body 14. The DLU 100 includes a cartridge assembly 200 housing a plurality of surgical fasteners or staples 223 (FIG. 2) and an anvil assembly 300 movably secured in relation to the cartridge assembly 200. The handle assembly 12 includes a stationary handle member 22, a movable handle member 24, and a barrel portion 26. An articulation lever 30 is mounted on the barrel portion 26 adjacent the rotatable member 28 to facilitate articulation of the DLU 100. A pair of knobs 32 is movably positioned along the barrel portion 26. The knobs 32 are advanced distally to approximate or close the cartridge assembly 200 and/or the anvil assembly 300, and are retracted proximally to unapproximate or open the cartridge assembly 200 and/or the anvil assembly 300. Actuation of the movable handle member 24 deploys lines of the staples 223 into tissue. In order to properly orient the cartridge and the anvil assemblies 200, 300 relative to the tissue to be stapled, the surgical stapling instrument 10 is additionally provided with a rotatable member 28 on the forward portion of the barrel portion 26. Rotation of the rotatable member 28 relative to the handle assembly 12 rotates the elongate body 14 and the DLU 100 relative to the handle assembly 12 so as to properly orient the cartridge assembly 200 and the anvil assembly 300 relative to the tissue to be stapled. For a detailed description of an exemplary surgical stapling instrument, reference may be made to U.S. Patent No. 7,128,253, the entire disclosure of which is hereby incorporated by reference herein.

As detailed above, the handle assembly 12 is a manually actuated handle assembly. It is envisioned that the DLU 100 may be part of an electromechanical or powered surgical system including a powered handle assembly and/or an adapter extending form the powered handle assembly that supports the DLU 100. Such a powered surgical system is described in U.S. Patent Publication No. 2014/0180000, the entire contents of which are hereby incorporated by reference.

With reference to FIG. 2, the cartridge assembly 200 includes a carrier 210 defining an elongated support channel 212. The elongated support channel 212 of the carrier 210 is dimensioned and configured to selectively receive a staple cartridge 220. The staple cartridge 220 defines retention slots 222 which each receive a fastener 223 and a pusher 226. A central longitudinal slot 234 is formed in and extends along the length of the staple cartridge 220 to facilitate passage of a knife blade of a drive bar 150 therethrough.

The anvil assembly 300 includes an anvil plate 310 having a plurality of staple deforming pockets/cavities (not explicitly shown) and a cover plate 320 secured to a top surface of anvil plate 310. The anvil assembly 300 further includes a knife blade 330 operatively interposed within the cavity defined between the anvil plate 310 and the cover plate 320.

During operation of the surgical stapling instrument 10, an actuation sled 228 translates through the staple cartridge 220 to advance upstanding cam wedges of the actuation sled 228 into sequential contact with pushers 226, to cause the pushers 226 to translate vertically within the retention slots 222 and to urge staples 223 from slots 222 into staple forming cavities of anvil plate 310 of the anvil assembly 300. A lower surface of the staple cartridge 220 defines longitudinal slots that are spaced apart from one another and that extend through the staple cartridge 220 to accommodate the cam wedges of the actuation sled 228.

For a detailed description of the construction and operation of an exemplary surgical stapling apparatus, cartridge assembly, and/or anvil assembly 300 reference may be made to U.S. Patent No. 8,011,555, the entire contents of which are hereby incorporated herein by reference.

Continuing to refer to FIG. 2, the cartridge assembly 200 includes a surgical cartridge buttress 500 operatively secured to an upper surface of the staple cartridge 220, by sutures "S1", "S2", to overlie at least some of the retention slots 222 and/or at least a portion of a length of the longitudinal slot 234. A first suture "S1" is threaded through a distal pair of recesses or attachment points 238 and around/over distal portion of cartridge buttress 500 and a second suture "S2" is threaded through a proximal pair of recesses or attachment points 236 and around/over proximal portion of cartridge buttress 500. A first end of each suture "S1", "S2" may be anchored or fixed in a respective one recesses of the proximal and distal pair of recesses or attachment points 236, 238 while a second end of each suture "S1", "S2" passes transversely across respective distal and proximal portions of cartridge buttress 500 and is anchored or fixed in a respective other recess of the proximal and distal pair of recesses or attachment points 236, 238.

The anvil plate 310 defines a proximal pair of recesses or attachment points 316 formed near a proximal end of the anvil plate 310 and disposed, one each, on opposed sides of the longitudinal slot 314. The anvil plate 310 defines a distal pair of recesses or attachment points 318 formed near a distal end of the anvil plate 310 and disposed, one each, on opposed sides of the longitudinal slot 314. At least one recess of the proximal pair of recesses or attachment points 316 and the distal pair of recesses or attachment points 318 is in the form of a slot or notch having a constricting profile so as to frictionally engage and/or pinch a suture, e.g., suture "S3" or "S4". The anvil assembly 300 further includes a surgical anvil buttress 600.

The anvil buttress 600 is secured to a lower surface of anvil plate 310, by sutures"S3", "S4", to overlie at least some of the anvil pockets and/or at least a portion of a length of the longitudinal slot 314. In particular, the suture "S3" is threaded across a distal portion of the anvil buttress 600 and each of a corresponding distal pair of recesses or attachment points 318, and the suture "S4" is threaded across a proximal portion of the anvil buttress 500 and each of a corresponding proximal pair of recesses or attachment points 316.

The cartridge buttress 500 and/or the anvil buttress 600 may be pre-loaded, e.g., from a manufacturer, onto the cartridge assembly 200 or the anvil assembly 300, respectively. Additional or replacement buttresses for the cartridge assembly 200 and/or the anvil assembly 300 may be secured as needed or desired. It is contemplated that the cartridge buttress 500 and/or the anvil buttress 600 can be attached by other means. For example, the buttresses 500, 600 can be attached using adhesives, welding, and/or attachment features incorporated in the buttress material.

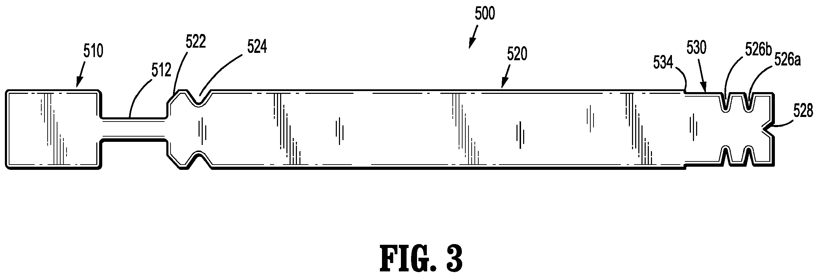

With reference to FIG. 3, various aspects of the cartridge buttress 500 are described below in accordance with the present disclosure. It will be appreciated that the anvil buttress 600 is substantially similar to the cartridge buttress 500. For this reason, the anvil buttress 600 will not be described in detail. As detailed above, the DLU 100 includes both a cartridge buttress 500 and an anvil buttress 600; however, it is contemplated that the DLU 100 may include only a cartridge buttress 500 or only an anvil buttress 600.

The buttress 500 has a uniform thickness and includes a head portion 510, a neck portion 512, a body portion 520, and a tail portion 530. The neck portion 512 extends from the head portion 510 to the body portion 520 to interconnect the head and body portions 510, 520. The tail portion 530 extends proximally from the body portion 520.

The buttress 500 is configured to be detachably secured to any size cartridge assembly 200. The body portion 520 defines a pair of opposing distal recesses 524 on transverse edges in a distal section 522 of the body portion 520. The pair of opposing distal recesses 524 may be utilized to secure body portion 520 to a distal region of the cartridge assembly 200, e.g., through a use of the suture "S1". The distal section 522 of the body portion 520 has a reduced transverse dimension, e.g., angled, arcuate, so as to be suitable for various shapes of cartridge assemblies.

The tail portion 530 of the buttress 500 defines a pair of opposing proximal recesses 526a formed therein. The pair of the opposing proximal recesses 526a is disposed on a transverse side of tail portion 530 near the proximal edge thereof. The opposing proximal pair of recesses 526a detachably secures the tail portion 530 of the buttress 500 to a proximal region of the cartridge assembly 200. The pair of opposing proximal recesses 526a may be substantially v-shaped.

To accommodate various cartridge assemblies, the tail portion 530 of the buttress 500 may include multiple pairs of opposing proximal recesses 526a, e.g., a first pair of opposing proximal recesses 526a and a second pair of opposing proximal recesses 526b (located distal of the first pair of opposing proximal recesses 526a). For example, when buttress 500 is to be used with a relatively long cartridge assembly 200, then the suture "S2" is extended across the tail portion 530 of buttress 500, passed through the first pair of opposing proximal recesses 526a of the buttress 500, and secured to the recesses 236 of the cartridge assembly 200. Alternatively, when buttress 500 is to be used with a relatively short cartridge assembly 200, then the suture "S2" is extended across the tail portion 530 of buttress 500, passed through the second pair of opposing proximal recesses 526b of the buttress 500, and secured to respective recess 316 of anvil assembly 300 and/or recesses 236 of cartridge assembly 200. Additionally, the buttress 500 may be secured to the cartridge assembly 200 with a suture, e.g., suture "S2" passed through each pair of proximal recesses 526a, 526b.

As detailed below, when the staples 223 are fired or ejected from the staple cartridge 220, each of the staples 223 pierce through the buttresses 500, 600 such that the staples 223 are captured in the buttresses 500, 600. The buttresses 500, 600 may also reinforce and seal staple lines formed by the staples 223. The buttresses 500, 600 may be utilized to retard or prevent tissue ingrowth from surrounding tissues thereby acting as an adhesion barrier and preventing the formation of unwanted scar tissue.

The buttresses 500, 600 may be formed of one or more layers. At least one of the layers of the buttresses 500, 600 is formed of a material which permits the staples 223 to penetrate through the layer of the respective buttress 500, 600 and resists tearing after the staples 223 pass through the layer. The buttresses 500, 600 may be formed from bioabsorbable or non-bioabsorbable materials. It should be understood that any combination of natural, synthetic, bioabsorbable, and non-bioabsorbable materials may be used to form the buttress material. The buttress material may be porous or non-porous, or a combination of porous and non-porous layers. The buttresses 500, 600 may also include layers or coatings including mendicants to be delivered to tissue surrounding the buttresses 500, 600. The mendicants may be released when the staples 223 penetrate through the respective buttress 500, 600 and/or may be released over a period of time after the staples penetrate through the respective buttress 500, 600.

For additional materials that may form one or more layers of the buttresses 500, 600, references can be made to commonly assigned U.S. Patent Nos. 5,542,594; 5,908,427; 5,964,774; 6,045,560; 7,823,592; and 7,938,307 and U.S. Patent Publication No. 2015/0231409, the entire contents of each are hereby incorporated by reference.

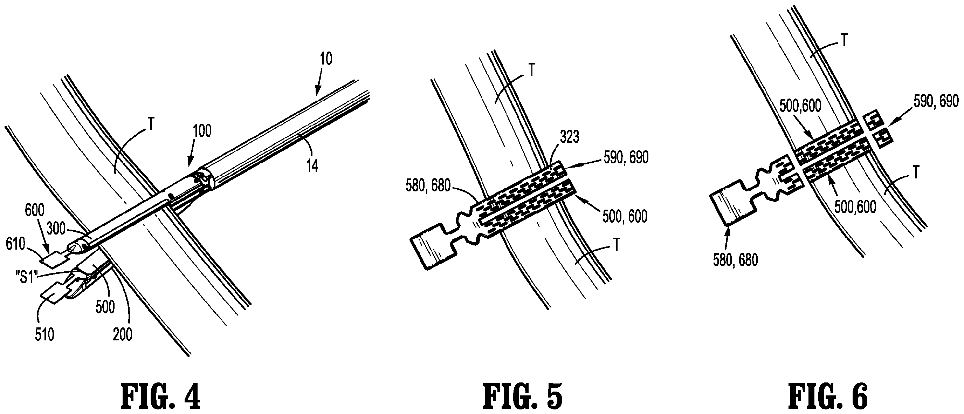

Referring to FIGS. 4-6, the use of the surgical stapling apparatus 10 is described in accordance with the present disclosure. Initially referring to FIG. 4, the surgical stapling apparatus 10 is provided with the DLU 100 coupled to a distal end of the elongated body 14 of the surgical stapling apparatus 10, and with the cartridge and anvil buttresses 500, 600 loaded onto the cartridge assembly 200 and the anvil assembly 300, respectively. It is contemplated that the cartridge and anvil buttresses 500, 600 may be loaded by a clinician before or during a surgical procedure. The surgical stapling apparatus 10 is manipulated to clamp tissue between the cartridge and anvil assemblies 200, 300.

Once the cartridge assembly 200 and the anvil assembly 300 are clamped onto tissue, the surgical stapling apparatus 10 is fired. In firing the surgical stapling apparatus 10, a drive bar (not shown) is advanced from a proximal-most position to a distal-most position of the DLU 100. In so doing, the sled 228 (FIG. 2) is advanced to fire staples 223 (FIG. 2) through the buttresses 500, 600 and the tissue. In addition, a knife blade of drive bar enters the buttresses 500, 600 thereby facilitating the dividing of the buttress 500, 600 between staple lines formed by the staples 223. As the drive bar begins to travel distally, the knife blade substantially simultaneously cuts through a central section of the proximal sutures "S2" and "S4" (FIG. 2) of the cartridge assembly 200 and the anvil assembly 300, thereby respectively freeing the proximal ends of the cartridge and anvil buttresses 500, 600. As the knife blade is moved distally, the knife blade slices or cuts longitudinally through both the cartridge and anvil buttresses 500, 600, thereby dividing the buttresses 500, 600 substantially in half.

Additionally, as the drive bar approaches the distal-most position, the drive bar and/or the knife blade engage a suture cutting assembly or suture release assembly to thereby sever or release distal sutures "S1" and/or "S3" and thus release a distal end of the buttresses 500, 600. For an exemplary suture cutting assembly reference can be made to U.S. Patent No. 8,011,555, the entire contents of which are hereby incorporated by reference.

With particular reference to FIG. 5, after the staples 223 are formed, the cartridge and anvil assemblies 200, 300 are transitioned towards the spaced-apart position such that the tissue is released from between the cartridge and anvil assemblies 200, 300. The surgical stapling apparatus 10 is then removed from over the tissue such that the buttresses 500, 600 and formed staples 223 remain in the tissue. As shown, each of the formed staples 223 passes through and is retained by the buttresses 500, 600 such that each of the staples 223 is captured in the buttresses 500, 600. The buttresses 500, 600 capture every staple 223 whether the staple 223 passes through tissue or if the staple 223 is a "remnant" staple which does not pass through tissue.

As shown in FIG. 6, after the surgical stapling apparatus 10 is removed from over the tissue, a clinician grasps a head portion of one or both of the buttresses 500, 600, e.g., head portion 510 of the cartridge buttress 500, and uses a cutting instrument (not shown) to cut through portions 580, 680 of the buttresses 500, 600 that extend beyond the tissue. The portions 580, 680 are then removed from the surgical site. The portions 580, 680 of the buttresses 500, 600 include "remnant" staples 223 which were captured during the firing of the surgical stapling apparatus 10.

Additionally or alternatively, a clinician grasps a tail portion 590, 690 of one or both of the buttresses 500, 600, e.g., tail portion 530 of the cartridge buttress 500, and uses the cutting instrument (not shown) to cut through portions 590, 690 of the buttresses 500, 600 that extend beyond the tissue. The portions 590, 690 are then removed from the surgical site. The portions 590, 690 of the buttresses 500, 600 include "remnant" staples 223 which were captured during the firing of the surgical stapling apparatus 10.

It is contemplated that the buttresses 500, 600 may only extend beyond tissue adjacent the head portion 510 or the tail portion 530. During such procedures, only the portions of the buttresses 500, 600 extending beyond the tissue adjacent the head portions, e.g., portions 580, 680, or the tail portions, e.g., portions 590, 690, are cut and removed from the surgical site.

After the portions 580, 680 and/or 590, 690 of the buttresses 500, 600, including any "remnant" staples 223, are removed from the surgical site, the buttresses 500, 600 and remaining staples 223 are disposed in tissue to seal the tissue along the staple lines formed by the staples 223 and/or to treat tissue. It will be appreciated that by capturing any "remnant" staples 223 in the portions 580, 590, 680, 690 of the buttresses 500, 600, the buttresses 500, 600 prevent any "remnant" staples 223 from remaining in the body cavity. This may be particularly useful when the staples 223 are coated with a mendicant or emit radioactivity. For example, during a brachytherapy procedure, radioactive staples are deployed in tissue along a resection margin such that the staples emit energy after being deployed to destroy cancer cells at the resection margin. Capturing any of the "remnant" radioactive staples prevents unintended exposure of tissue to the energy emitted from the radioactive staples.

The various embodiments disclosed herein may also be configured to work with robotic surgical systems and what is commonly referred to as "Telesurgery." Such systems employ various robotic elements to assist the surgeon and allow remote operation (or partial remote operation) of surgical instrumentation. Various robotic arms, gears, cams, pulleys, electric and mechanical motors, etc. may be employed for this purpose and may be designed with a robotic surgical system to assist the surgeon during the course of an operation or treatment. Such robotic systems may include remotely steerable systems, automatically flexible surgical systems, remotely flexible surgical systems, remotely articulating surgical systems, wireless surgical systems, modular or selectively configurable remotely operated surgical systems, etc.

The robotic surgical systems may be employed with one or more consoles that are next to the operating theater or located in a remote location. In this instance, one team of surgeons or nurses may prep the patient for surgery and configure the robotic surgical system with one or more of the instruments disclosed herein while another surgeon (or group of surgeons) remotely control the instruments via the robotic surgical system. As can be appreciated, a highly skilled surgeon may perform multiple operations in multiple locations without leaving his/her remote console which can be both economically advantageous and a benefit to the patient or a series of patients.

The robotic arms of the surgical system are typically coupled to a pair of master handles by a controller. The handles can be moved by the surgeon to produce a corresponding movement of the working ends of any type of surgical instrument (e.g., end effectors, graspers, knifes, scissors, etc.) which may complement the use of one or more of the embodiments described herein. The movement of the master handles may be scaled so that the working ends have a corresponding movement that is different, smaller or larger, than the movement performed by the operating hands of the surgeon. The scale factor or gearing ratio may be adjustable so that the operator can control the resolution of the working ends of the surgical instrument(s).

The master handles may include various sensors to provide feedback to the surgeon relating to various tissue parameters or conditions, e.g., tissue resistance due to manipulation, cutting or otherwise treating, pressure by the instrument onto the tissue, tissue temperature, tissue impedance, etc. As can be appreciated, such sensors provide the surgeon with enhanced tactile feedback simulating actual operating conditions. The master handles may also include a variety of different actuators for delicate tissue manipulation or treatment further enhancing the surgeon's ability to mimic actual operating conditions.

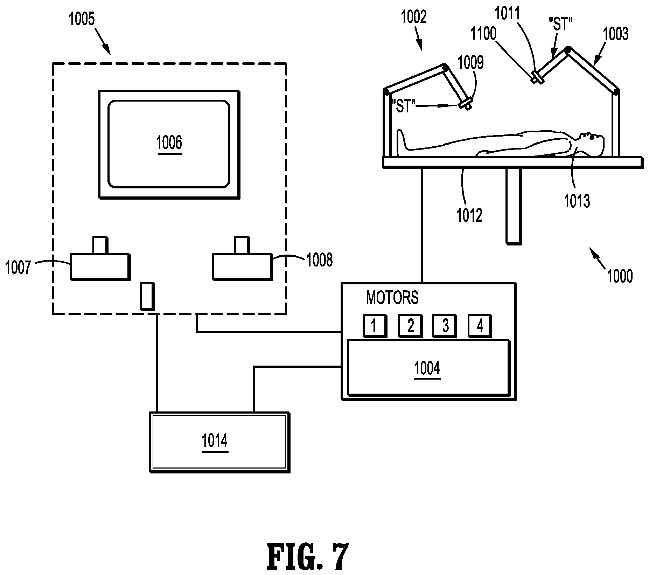

Turning to FIG. 7, a robotic surgical system configured for use in accordance with the present disclosure is shown generally identified by reference numeral 1000. Aspects and features of robotic surgical system 1000 not germane to the understanding of the present disclosure are omitted to avoid obscuring the aspects and features of the present disclosure in unnecessary detail.

Robotic surgical system 1000 generally includes a plurality of robot arms 1002, 1003; a control device 1004; and an operating console 1005 coupled with control device 1004. Operating console 1005 may include a display device 1006, which may be set up in particular to display three-dimensional images; and manual input devices 1007, 1008, by means of which a person, e.g., a surgeon, may be able to telemanipulate robot arms 1002, 1003 in a first operating mode. Robotic surgical system 1000 may be configured for use on a patient 1013 lying on a patient table 1012 to be treated in a minimally invasive manner. Robotic surgical system 1000 may further include a database 1014, in particular coupled to control device 1004, in which are stored, for example, preoperative data from patient 1013 and/or anatomical atlases.

Each of the robot arms 1002, 1003 may include a plurality of members, which are connected through joints, and an attaching device 1009, 1011, to which may be attached, for example, a surgical tool "ST." One or more of the surgical tools "ST" may include a DLU, e.g., DLU 100, similar to those detailed above, thus providing such functionality on a robotic surgical system 1000.

Robot arms 1002, 1003 may be driven by electric drives, e.g., motors, connected to control device 1004. Control device 1004, e.g., a computer, may be configured to activate the motors, in particular by means of a computer program, in such a way that robot arms 1002, 1003, their attaching devices 1009, 1011, and, thus, the surgical tools "ST" execute a desired movement and/or function according to a corresponding input from manual input devices 1007, 1008, respectively. Control device 1004 may also be configured in such a way that it regulates the movement of robot arms 1002, 1003 and/or of the motors.

While several embodiments of the disclosure have been shown in the drawings, it is not intended that the disclosure be limited thereto, as it is intended that the disclosure be as broad in scope as the art will allow and that the specification be read likewise. Any combination of the above embodiments is also envisioned and is within the scope of the appended claims. Therefore, the above description should not be construed as limiting, but merely as exemplifications of particular embodiments. Those skilled in the art will envision other modifications within the scope of the claims appended hereto.

The invention may be described by reference to the following numbered paragraphs:-
1. A method of stapling tissue comprising:
   clamping tissue between a cartridge assembly and an anvil assembly of a loading unit;
   firing the loading unit such that fasteners secured in the cartridge assembly pass through a body portion of a first buttress positioned between the clamped tissue and one of the cartridge assembly or the anvil assembly, the first buttress capturing fasteners fired from a portion of the cartridge assembly extending beyond the tissue;
   trimming a first portion of the first buttress adjacent a first edge of the tissue; and
   removing the first portion of the first buttress, including at least one remnant fastener, from a surgical site.
2. The method according to paragraph 1, wherein trimming the first portion of the first buttress adjacent the first edge of the tissue includes trimming the first portion of the first buttress adjacent a distal end of the first buttress.
3. The method according to paragraph 1, wherein trimming the first portion of the first buttress adjacent the first edge of the tissue includes trimming the first portion of the first buttress adjacent a proximal end of the first buttress.
4. The method according to paragraph 2, further comprising:
   trimming a second portion of the first buttress adjacent a second edge of the tissue adjacent a proximal end of the first buttress; and
   removing the second portion of the first buttress, including at least one remnant fastener, from the surgical site.
5. The method according to paragraph 1, wherein firing the loading unit includes the fasteners piercing a body portion of a second buttress positioned between the clamped tissue and the anvil assembly, the first buttress being positioned between the clamped tissue and the cartridge assembly.
6. The method according to paragraph 5, further comprising trimming a first portion of the second buttress adjacent the first edge of the tissue.
7. A loading unit comprising:
   a cartridge assembly having a first tissue contacting surface and fasteners disposed therein;
   an anvil assembly having a second tissue contacting surface, the cartridge and anvil assemblies having a first position in which the first and second tissue contacting surfaces are spaced apart from one another and a second position in which at least one of the first and second tissue contacting surfaces are approximated relative to the other to clamp tissue therebetween;
   a first buttress operatively secured to one of the first or second tissue contacting surfaces; and
   a sled translatable through the cartridge assembly to fire the fasteners from the cartridge assembly towards the anvil assembly, the fasteners configured to pass through the first buttress and form upon contact with the anvil, the first buttress configured to capture at least one of the formed fasteners.
8. The loading unit according to paragraph 7, wherein the first buttress is configured to capture each formed fastener.
9. The loading unit according to paragraph 7, wherein the sled is configured to sequentially fire the fasteners.
10. The loading unit according to paragraph 7, wherein the fasteners are radioactive fasteners configured to emit energy to tissue.
11. The loading unit according to paragraph 7, wherein the first buttress is operatively secured to the first tissue contacting surface.
12. The loading unit according to paragraph 11, further comprising a second buttress operatively secured to the second tissue contacting surface, the fasteners configured to pass through the second buttress and form upon contact with the anvil, the second buttress configured to capture each formed fastener.
13. The loading unit according to paragraph 12, wherein the first buttress is secured to the first tissue contacting surface.
14. A surgical instrument, comprising:
   a handle assembly;
   an elongate body extending from the handle assembly; and
   a loading unit supported by a distal portion of the elongate body, the loading unit including:
      a cartridge assembly having a first tissue contacting surface and fasteners disposed therein;
      an anvil assembly having a second tissue contacting surface, the cartridge and anvil assemblies having a first position in which the first and second tissue contacting surfaces are spaced apart from one another and a second position in which at least one of the first and second tissue contacting surfaces are approximated relative to the other to clamp tissue therebetween;
      a first buttress operatively secured to one of the first or second tissue contacting surfaces; and
      a sled translatable through the cartridge assembly to fire the fasteners from the cartridge assembly towards the anvil assembly, the fasteners configured to pass through the first buttress and form upon contact with the anvil, the first buttress configured to capture at least one of the formed fasteners.
15. The surgical instrument according to paragraph 14, wherein the handle assembly is manually actuated.

## Claims

1. A loading unit comprising:
a cartridge assembly having a first tissue contacting surface and fasteners disposed therein;
an anvil assembly having a second tissue contacting surface, the cartridge and anvil assemblies having a first position in which the first and second tissue contacting surfaces are spaced apart from one another and a second position in which at least one of the first and second tissue contacting surfaces are approximated relative to the other to clamp tissue therebetween;
a first buttress operatively secured to one of the first or second tissue contacting surfaces; and
a sled translatable through the cartridge assembly to fire the fasteners from the cartridge assembly towards the anvil assembly, the fasteners configured to pass through the first buttress and form upon contact with the anvil, the first buttress configured to capture at least one of the formed fasteners.

2. The loading unit according to claim 1, wherein the first buttress is configured to capture each formed fastener.

3. The loading unit according to claim 2, wherein the sled is configured to sequentially fire the fasteners.

4. The loading unit according to any preceding claim, wherein the fasteners are radioactive fasteners configured to emit energy to tissue.

5. The loading unit according to any preceding claim, wherein the first buttress is operatively secured to the first tissue contacting surface.

6. The loading unit according to claim 5, further comprising a second buttress operatively secured to the second tissue contacting surface, the fasteners configured to pass through the second buttress and form upon contact with the anvil, the second buttress configured to capture each formed fastener.

7. The loading unit according to claim 6, wherein the first buttress is secured to the first tissue contacting surface.

8. A surgical instrument, comprising:
a handle assembly;
an elongate body extending from the handle assembly; and
a loading unit according to any preceding claim supported by a distal portion of the elongate body, the loading unit including:
a cartridge assembly having a first tissue contacting surface and fasteners disposed therein;
an anvil assembly having a second tissue contacting surface, the cartridge and anvil assemblies having a first position in which the first and second tissue contacting surfaces are spaced apart from one another and a second position in which at least one of the first and second tissue contacting surfaces are approximated relative to the other to clamp tissue therebetween;
a first buttress operatively secured to one of the first or second tissue contacting surfaces; and
a sled translatable through the cartridge assembly to fire the fasteners from the cartridge assembly towards the anvil assembly, the fasteners configured to pass through the first buttress and form upon contact with the anvil, the first buttress configured to capture at least one of the formed fasteners.

9. The surgical instrument according to claim 8, wherein the handle assembly is manually actuated.

10. A surgical instrument according to claim 8 or claim 9 for use in stapling tissue.
